# EUROPEAN PATENT APPLICATION

(11) **EP 4 053 260 A1**
(43) Date of publication of application: **07.09.2022**
(21) Application number: 21160739.5
(22) Date of filing: 04.03.2021
(51) Int. Cl.: C12M 1/06, C12M 1/34, C12N 13/00

(54) **DEVICE WITH AN INSERT FOR TREATING CELL MATERIAL**

(71) Applicant: Bühler AG, 9240 Uzwil (CH)
(72) Inventor: Buchmann, Leandro, 8408 Winterthur (CH)
(74) Representative: Hepp Wenger Ryffel AG

(57) **Abstract**

The present invention is related to an insert (4) for a device (1) for treating cells, wherein said insert (4) comprises electrodes (2a, 2b) for emitting electric pulses and has an electrical connection (4a), wherein a treatment space (3) is provided in said insert (4), wherein said treatment space (3) can be penetrated by the electric pulses emitted by said electrodes (2a, 2b) and an electric field resulting therefrom. The present invention is also related to such a device (1) and to a method performed with said device (1).

## Description

The present invention is related to a device for treating cells for medical, environmental, food applications, and bio-based industries (including yeast, lactobacilli, algae, and cell tissue production systems), in particular including targeted inactivation, the extraction of bioactive compounds, and the stimulation of cell growth and/or cellular compounds, wherein said treatment is conducted in a treatment space provided in an insert, and to a method for treating cells in such a device.

It is known that prokaryotic and eukaryotic cells are influenced by the application of electric fields. Stimulation of cell growth, as well as cell death, inactivation of microorganisms, or specific extraction of cell constituents can occur, depending on the applied electric field strength (e.g. Buchmann L and Mathys A (2019), Perspective on Pulsed Electric Field Treatment in the Bio-based Industry, Front. Bioeng. Biotechnol. 7:265, doi: 10.3389/fbioe.2019.00265).

EP-2 308 969 B1 describes a PEF (pulsed electric field) method where a cell material suspended in an electrically conductive liquid, the cell material being positioned between two electrodes, by exposure of 1 to 100 electric field strength pulses, such that a voltage increase takes place between the two electrodes of 10% to 90% of a target voltage of the electric field strength pulses within a period of 0.1 ns to 100 ns, the electric field strength pulses have a pulse duration of 5 ns to 5000 ns, and the electric field strength pulses, upon reaching the target voltage, have an electric field strength of 0.5 kV/cm to 50 kV/cm, showed an accelerated cell proliferation and/or increased formation of cellular constituents.

In the device for treating cell material used in EP 2 308 969 B1, an electroporation cuvette having the suspension of cell material described above was continuously pumped through an arrangement of two electrodes and exposed to an electric field in an electrically conductive liquid (paragraph [0020] of EP-2 308 969 B1).

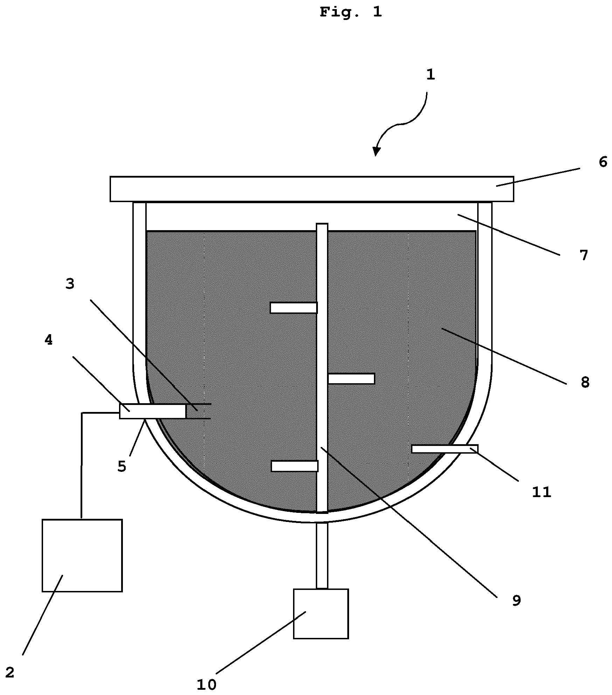

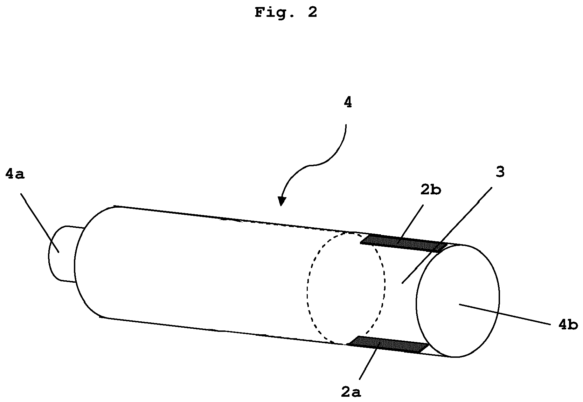

As an alternative, it has been suggested to provide the material to be treated in a vessel (also called bioreactor), and for treatment transfer the material in a line that is connected with an outlet of said vessel. The other end of said line is connected with an inlet into said vessel, so that the vessel and the line form a fluid circuit. Said line is also called bypass (see e.g. Buchmann L and Mathys A (2019), Perspective on Pulsed Electric Field Treatment in the Bio-based Industry, Front. Bioeng. Biotechnol. 7:265, doi: 10.3389/fbioe.2019.00265 (Fig.1); Gal-van-D'Alessandro et al., Fermentation Assisted by Pulsed Electric Field and Ultrasound: A Review, Fermentation 2018, 4, 1; doi:10.3390 (Fig. 2); DE 10 2010 019 937 A1).

As a further alternative, it has been suggested to carry out a treatment of cell material with electric pulses in a bioreactor. For this purpose, electrodes for emitting the electric pulses are arranged directly in the bioreactor, optionally along with a mixing unit (e.g. US-9,096,847 for inducing metabolic activity of treated cell material). However, such a device suffers from a problem of inhomogeneous process conditions. The mixing unit is not capable of providing reliably homogenous conditions in the entire bioreactor, and furthermore interferes with the electric pulses emitted by the electrodes. This is a problem since influencing the cell proliferation as described above depends on precise adherence to process conditions. With the device of US-9,096,847, it is not possible to obtain homogeneous process conditions in the entire reaction vessel and therefore also no homogeneously treated cell material. Furthermore, the device has high energy requirements, due to the necessity of consistently operating the mixing unit.

In US-2012/0040428 A1, a similar device is described. Here, it is suggested to provide the electrodes in a housing with openings. The material to be treated flows through these openings and is treated in a path having high electrical resistance between adjacent electrodes. The housing with the electrodes is permanently arranged in the bioreactor.

Especially for performing on-line analytical methods, the above devices are not optimal.

It was therefore the object of the present invention to provide a device and a method for treating cells in an efficient and cost-effective manner.

The above problem has been solved by the present invention.

In detail, the present invention is related to an insert for a device for treating cells, wherein said insert comprises electrodes for emitting electric pulses and has an electrical connection, characterized in that a treatment space is provided in said insert, wherein said treatment space can be penetrated by the electric pulses emitted by said electrodes and an electric field resulting therefrom.

According to the present invention, an insert of a device for treating cells is provided, wherein said insert can be inserted through an opening in the device into the inner space of the device. In the inner space of the device, cell material can be provided, which then comes into contact with the insert when being moved through the opening of the device.

According to the present invention, the device and the insert are separate components which during operation are combined with each other. This allows easy and reliable cleaning or sterilization of the insert, for example in an autoclave.

The treatment space provided in the insert is relatively small as compared to the inner space of the device, so that homogenous conditions are established in the treatment space during cell treatment operation.

After the operation has been terminated, the treated cells can be taken from the device through a sampling port (e.g. the inlet) of the device, or in an embodiment described below by maintaining the cells in a treatment space in the insert and removing the insert from the device. In this way, an easy analysis of the treated material is possible.

The insert may have any shape that is suitable for the purposes of the present invention, e.g. cylindrical or cuboid shape. A cylindrical shape is preferred.

The insert may be made of any material that is suitable for the purposes of the present invention, e.g. from a metallic material or a plastic material.

According to a preferred embodiment of the present invention, the insert is a static insert. This means that the insert is not moved once it is positioned in the device of the invention described below.

The insert of the present invention is provided with a treatment space. According to the present invention, said treatment space is an area into which the material to be treated can enter and in which said material can be treated with electric pulses.

According to one embodiment of the present invention, said treatment space is an open area between electrodes that are provided at one end of the insert. In other words, electrode and preferably two electrodes protrude, preferably parallel to one another, from an end of the insert, so that an area between these electrodes is formed. Said area is open and only limited by the electrodes and the end of the insert from which the electrodes protrude. Accordingly, in this embodiment the treatment space is fully accessible by material to be treated. Preferably, the treatment space is limited to the area between the electrodes, and the electrodes themselves are isolated (e.g. with a non-conductive material).

According to another embodiment of the present invention, said treatment space is a chamber at one end of the insert, preferably a cylindrical chamber. The insert has an opening through which the material to be treated can enter into said treatment space provided within said insert. Preferably, the opening allowing entrance into said chamber is provided in an end face (top face or bottom face) of the insert. Most preferably, the entire end face of said insert is open, allowing good access to the chamber constituting the treatment space.

In this embodiment according to the present invention, the opening in the insert to the treatment space (in the chamber at one end of the insert) may be closed by usual means (e.g. a door or flap or a cap).

The insert according to the present invention furthermore comprises two or more, preferably two electrodes for emitting electrical pulses into the treatment space. Alternatively, two plates e.g. of a capacitor may be used for this purpose. The electrodes and the treatment space are arranged such that said treatment space can be penetrated by the electric pulses emitted by said electrodes and an electric field resulting therefrom.

According to a preferred embodiment of the present invention, said treatment space is arranged between said electrodes or plates so that the treatment space can be penetrated by the emitted electric pulses and an electric field resulting therefrom. According to this embodiment, said electrodes or plates e.g. of a capacitor are arranged parallel to each other, opposite each other having a distance suitable for the generation of adequate electric fields. Such arrangements are well known and need not be explained in detail here.

According to a preferred embodiment, said treatment space is a chamber at one end of the insert, as described above, and the electrodes or plates e.g. of a capacitor are arranged parallel to each other at the bottom and top surface of said treatment space.

According to another preferred embodiment of the present invention, said treatment space is an open area between electrodes that protrude, preferably parallel to one another, from an end of the insert, so that an area between these electrodes is formed.

Said electrodes or plates e.g. of a capacitor are electrically connected with a unit for generating electric pulses. For this purpose, the insert according to the present invention comprises an electrical connection to which electrical lines from said unit for generating electric pulses can be connected. Such electrical connections are known and do not have to be discussed in detail here.

Preferably, at one end of said insert there is provided an electrical connection, and at the other end of said insert there is provided said treatment space.

According to another preferred embodiment of the present invention, said insert is a sensor. According to this embodiment, the portion of the insert that is not occupied by the treatment space comprises the necessary components of a respective known sensor. Typical sensors are pH sensors, conductivity sensors, temperature sensors, or ion sensors. According to the present invention any conventional sensor type may be used.

As discussed above, the insert of the present invention can be easily attached to or removed from a device for treating cells. In the device as such, typically the cells are cultivated and/or proliferated. In the device with the insert described above, the cells are treated in the provided treatment space using electric pulses.

Accordingly, the present invention is also related to a device, comprising an inlet and an inner space, and further comprising an opening and a unit for generating electric pulses, characterized in that the device further comprises an insert according to the present invention, that can be inserted in said opening.

According to the present invention, the device may be any unit with an inner space in which a fluid can be stored. With the device of the present invention, typically biological material in the form of a suspension is treated. Accordingly, the device may be a unit for taking up and storing such a suspension. For example, said device may be a cylindrical tank with at least one inlet suitable for inserting (and discharging) a suspension.

Preferably, said device may be a bioreactor. A bioreactor is generally known in the art. A bioreactor is a device or system that supports a biologically active environment. Preferably, a bioreactor is a vessel in which a (bio)chemical process can be carried out which involves organisms or biochemically active substances derived from such organisms. This process can either be aerobic or anaerobic, for example fermentation. Conventional bioreactors range in size from litres to cubic metres, and are often made of stainless steel.

According to a preferred embodiment of the present invention, said device may comprise one or more lines arranged at said device. According to the present invention, any line conventionally used for transporting a fluid may be used. Preferably, the lines are pipes, for example made from stainless steel, tubes or hoses.

According to the present invention, said device has an inlet for allowing insertion of a fluid into said device. According to a preferred embodiment, the top face of the device is designed as a cap or a valve that can be opened or closed, thus allowing inserting and discharging a fluid into or from the device. In this embodiment, the material introduced into the device is circulated within the device by a stirrer or mixer, or by external actuating means such as in known wave mix bioreactors.

According to another embodiment of the present invention, the device may comprise an inlet in a top face of said device, which may be connected to one line for transferring a fluid to said device, and an outlet which is formed in a bottom face of said device and which may be connected to another line for transferring a fluid away from said device. While it is principally possible to provide an inlet and/or outlet of said device for taking up a fluid also in a side wall of said device, providing said inlet and said outlet in a top face and bottom face of said unit is preferred for establishing a closed circuit in which the biological material to be treated can be circulated.

Preferably, at said inlet and/or said outlet units for regulating the flow may be provided. As an example, conventionally used valves or locks may be mentioned.

Said device comprises an opening into which the insert according to the present invention can be inserted, so that the insert comes into contact with the inner space of the device and any fluid present in said inner space. Said opening has a shape that corresponds to the shape of the insert of the invention. Known standard fixing elements such as clips or threads may be provided for preventing unwanted separation of the insert form the device. When the insert is not positioned in the opening, the opening should be sealed in order to prevent any leakage of fluid from the inner space through the opening.

The device according to the present invention comprises a unit for generating electric pulses. Such units are generally known. By way of example, cable pulse generators, semiconductor-based pulse generators, or relaxation oscillators can be mentioned.

The generated electric pulses are emitted into the treatment space. This is preferably done by two or more electrodes or plates e.g. of a capacitor, as described above, which are electorally connected with said unit for generating electric pulses.

The electric field to be applied to the treatment space must be characterized such that it provides for the desired effect, e.g. that it stimulates the growth of the treated cells. Corresponding electric fields are known from the prior art, for example from EP-2 308 969 B1. According to the present invention, an electric field generated from such electric pulses can be used, such that a voltage increase takes place between the two electrodes or plates of a capacitor of the device of 10% to 90% of a target voltage of the electric pulses within a period of 0.1 ns to 1000 ns, the electric pulses have a pulse duration of 5 ns to 50000 ns, and the electric pulses, upon reaching the target voltage, have an electric field strength of 0.5 kV/cm to 100 kV/cm.

According to the present invention, said device is suitable for medical, environmental, food applications, and bio-based industries (including yeast, lactobacilli, algae, and cell tissue production systems, in particular including targeted inactivation, the extraction of bioactive compounds, and the stimulation of cell growth and/or cellular compounds. These applications are described in Buchmann L and Mathys A (2019), Perspective on Pulsed Electric Field Treatment in the Bio-based Industry, Front. Bioeng. Biotechnol. 7:265, doi: 10.3389/fbioe.2019.00265.

According to the present invention, electric pulses are applied to a cell material located in the treatment space of the insert according to the present invention. As a result, the cell material is subjected to the electric pulses and treated.

According to the present invention, basically any material composed of at least one cell, that is, both eukaryotic and prokaryotic cells, can be used as the cell material to be treated. The cell material can be unicellular or multicellular organisms. Examples would be bacteria, yeasts, microalgae, plant cells, and fungal cells or their spores, mycelia, seeds or seedlings and somatic animal cells or germ cells and mammalian cells. Furthermore, multicellular tissues such as meristems in plants and epithelial or connective tissue in humans or animals can be treated.

The cell material is usually (but not necessarily) isolated and/or purified in a known manner before being treated according to the invention. Preferably, the cell material can already be propagated in a known manner in suitable and known culture media to a desired degree before the treatment according to the invention.

The cell material is preferably suspended in an electrically conductive liquid prior to the treatment according to the invention. Electrically conductive liquids are well known. According to the invention, it is necessary to use electrically conductive liquids which have no adverse effects on cell viability, that is, in particular, are non-toxic. According to the invention, water is preferably used as the electrically conductive liquid, wherein the water can be adjusted to a desired pH value by means of suitable and known additives. According to the invention, a pH value in the range of 6.0 to 14.0, preferably 7 to 12 is preferred. According to another preferred embodiment of the invention, macro- and micronutrients as well as trace elements can be added for supporting cultivation and/or proliferation of the cells. Such macro- and micronutrients as well as trace elements are known and can be selected by a skilled person depending on the kind of cells.

According to the present invention, the suspensions described above can be prepared in a conventional manner and stored until treatment. However, the suspensions can also be provided immediately before the treatment according to the invention.

The cell material or a suspension containing the cell material is passed through the opening of said treatment space which is located inside the insert according to the present invention. This may be either achieved by passive diffusion of the fluid present in the inner space of said device of the invention through said opening, or preferably with the aid of a mixing unit that distributes the fluid homogeneously in the inner space of the device of the invention.

According to this present invention, any mixing unit conventionally used in the field of the invention may be considered.

While the mixing unit could be principally operated manually, it is preferred that the mixing unit is operated by a motor. According to this present invention, any motor conventionally used in the field of the invention may be considered.

According to a preferred embodiment of the present invention, the device of the invention may comprise one or more additional sensors. Typical sensors are pH sensors, conductivity sensors, temperature sensors, permittivity sensors or ion sensors. According to the present invention any conventional sensor type may be used.

As discussed above, the treatment space provided in the insert according to the present invention is relatively small as compared to the inner space of the device, so that homogenous conditions are established in the treatment space during cell treatment operation. According to a preferred embodiment of the present invention, the treatment space provided in said insert has a volume that is in the range of 0.1% to 10%, preferably 1% to 5%, of the volume of the inner space of said device.

The present invention is furthermore related to a method for treating cells for stimulating cell growth, performed in a device of the invention, comprising the steps
a) introducing cell material through the inlet of the device into the inner space of the device,
b) passing of the cell material into the treatment space and subjecting it to the electric pulses penetrating the treatment space.

The method can be performed as already described above. As stated above, it is preferred that the cell material is provided as a suspension in an electrically conductive liquid.

As stated above, it is further preferred that an electric field is applied with such electric field strength pulses, so that a voltage increase takes place between the two electrodes (6a, 6b) or plates of 10% to 90% of a target voltage of the electric field strength pulses within a period of 0.1 ns to 1000 ns, the electric field strength pulses have a pulse duration of 5 ns to 50000 ns, and the electric field strength pulses, upon reaching the target voltage, have an electric field strength of 0.5 kV/cm to 100 kV/cm.

The treatment process of the present invention may be performed continuously or batch-wise. In the latter case, the device and insert of the present invention may be emptied before another treatment cycle is started.

Optionally, a sterilization/sanitization step may be performed prior to the start of another treatment cycle. Known sterilization/ sanitization processes such as steam-based processes can be used in accordance with the present invention.

According to a preferred embodiment of the present invention, the insert of the invention is sterilized (autoclaved) in-line, i.e. after it has been positioned in the opening of the device of the invention. In order to prevent any contamination, the insert should not be interchanged after said sterilization step of said insert.

After the insert has been positioned and fixed in the opening of the device, and optionally has been sterilized in-line, the inner space of the device is filled with the material to be treated, as described above. The cell material in the inner space of the device may be distributed by means of a mixing unit, as described above. A portion of said cell material enters the treatment space in the insert and is treated therein, as described above.

After the treatment/cultivation/production has been terminated, the cell material is discharged from the device, and the insert is removed or sterilized / sanitized in-line.

The present invention further relates to the use of the device or apparatus according to the present invention described herein for medical, environmental, food applications, and bio-based industries (including yeast, bacteria, microalgae, as well as plant or animal cells and cell tissue production systems, in particular targeting inactivation, the extraction of bioactive compounds, and/or the stimulation of cell growth and/or cellular compounds. These applications are described in Buchmann L and Mathys A (2019), Perspective on Pulsed Electric Field Treatment in the Bio-based Industry, Front. Bioeng. Biotechnol. 7:265, doi: 10.3389/fbioe. 2019.00265.

In particular, the insert and device of the invention may be used for analytical purposes, where only a small amount of cell material is to be treated.

The present invention is explained below by way of non-limiting examples and figures.
- Fig. 1: shows a schematic representation of an embodiment of the device of the present invention
- Fig. 2: shows a schematic representation of a first embodiment of the insert of the present invention
- Fig. 3: shows a schematic representation of a second embodiment of the insert of the present invention

In the figures, same references numbers designate the same components.

In Fig. 1, a schematic representation of an embodiment of the device 1 of the present invention is shown. Said device 1 comprises a unit 2 for generating electric pulses, which by means of electrodes 2a, 2b (see Fig. 2) are emitted into a treatment space 3 provided at one end of an insert 4. Said insert is positioned in an opening 5 of the device 1 and thus comes into contact with the inner space 7 of the device.

An inlet 6 (here in the form of a cap that can be opened or closed) provides access to the inner space 7 of the device. In said inner space, for example a cell material suspension 8 may be provided.

The device of Fig. 1 also comprises a mixing unit 9 for distributing the cell material suspension 8. The mixing unit 9 is operated with a motor 10, for example an electric motor.

The device of Fig. 1 furthermore comprises a sensor 11, e.g. a temperature sensor.

In Fig. 2, a schematic representation of a first embodiment of the insert 4 of the present invention is shown.

The insert 4 of Fig. 2 has a cylindrical shape and comprises, at its one end face, an electrical connection 4a for electrically connecting the insert with a unit 2 for generating electric pulses. In said insert 4, electric lines (not shown) are provided which guide the electric pulse from the electrical connection 4a to electrodes 2a, 2b.

The insert of Fig. 2 comprises a treatment space 3 in the form of a chamber at the end of the insert 4 remote from the electrical connection 4a. Said treatment space is provided between a wall (indicated with broken lines) separating the remaining portion of the insert 4 from the treatment space 3, and the other end face 4b of the insert 4. In the embodiment of Fig. 2, said end face 4b constitutes an opening allowing entrance into the treatment space 3.

Within said treatment space, in the embodiment of Fig. 2 at the bottom face and top face thereof, there are provided the electrodes 2a, 2b. The electrodes 2a, 2b are arranged parallel, and the treatment space 3 is provided between said electrodes 2a, 2b, so that it can be penetrated by the electric pulses emitted from said electrodes and the electric field resulting therefrom.

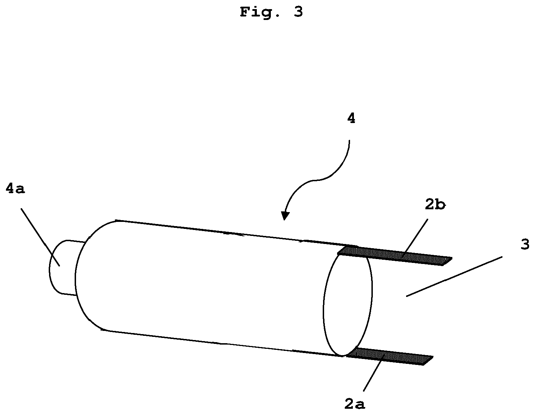

In Fig. 3, a schematic representation of a second embodiment of the insert 4 of the present invention is shown.

The insert 4 of Fig. 2 has a cylindrical shape and comprises, at its one end face, an electrical connection 4a for electrically connecting the insert with a unit 2 for generating electric pulses. In said insert 4, electric lines (not shown) are provided which guide the electric pulse from the electrical connection 4a to electrodes 2a, 2b.

The insert of Fig. 2 comprises a treatment space 3 provided as an open area between the electrodes 2a, 2b that protrude from the end of the insert 4 remote from the electrical connection 4a. The electrodes 2a, 2b are arranged parallel, and the treatment space 3 is provided between said electrodes 2a, 2b, so that it can be penetrated by the electric pulses emitted from said electrodes and the electric field resulting therefrom.

## Claims

1. Insert (4) for a device (1) for treating cells, wherein said insert (4) comprises electrodes (2a, 2b) for emitting electric pulses and has an electrical connection (4a), **characterized in that** a treatment space (3) is provided in said insert (4), wherein said treatment space (3) can be penetrated by the electric pulses emitted by said electrodes (2a, 2b) and an electric field resulting therefrom.

2. Insert according to claim 1, **characterized in that** the insert (4) is a static insert.

3. Insert according to claim 1 or 2, **characterized in that** the treatment space (3) is a chamber at one end of the insert (4) and has an opening (4b).

4. Insert according to claim 1 or 2, **characterized in that** said treatment space (3) is an open area between the electrodes (2a, 2b) that are provided at one end of the insert (4).

5. Insert according to claim 3, **characterized in that** the electrodes (2a, 2b) are provided in said chamber.

6. A device (1), comprising an inlet (6) and an inner space (7), and further comprising an opening (5) and a unit (2) for generating electric pulses, **characterized in that** the device (1) further comprises an insert (4) according to any of claims 1 to 5, that can be inserted in said opening (5).

7. The device according to claim 6, **characterized in that** the insert (4) is electrically connected to the unit (2) for generating electric pulses via the electrical connection (4a) of said insert (4).

8. The device according to claim 6 or 7, **characterized in that** the device (1) further comprises a mixing unit (9).

9. The device according to claim 8, **characterized in that** the mixing unit (9) is connected to a motor (10).

10. The device according to any of claims 6 to 9, **characterized in that** the device (1) further comprises at least one additional sensor (11).

11. The device according to any of claims 6 to 10, **characterized in that** the treatment space (3) provided in said insert (4) has a volume that is in the range of 0.1% to 10%, preferably 1% to 5%, of the volume of the inner space (7) of said device (1).

12. The device according to any of claims 6 to 11, **characterized in that** the unit (2) for generating and emitting electric pulses can generate electric pulses so that a voltage increase take place between the two electrodes (2a, 2b) of 10% to 90% of a target voltage of the electric pulses within a period of 0.1 ns to 1000 ns, the electric pulses have a pulse duration of 5 ns to 50000 ns, and the electric pulses, upon reaching the target voltage, have an electric field strength of 0.5 kV/cm to 100 kV/cm.

13. A method for treating cells for stimulating cell growth, performed in a device (1) according to any one of claims 6 to 12, comprising the steps
a) introducing cell material through the inlet (6) of the device (1) into the inner space (7) of the device (1),
b) passing of the cell material into the treatment space (3) and subjecting it to the electric pulses penetrating the treatment space (3).

14. The method according to claim 13, **characterized in that** the cell material is provided in the inner space (7) of said device (1) as a suspension (8) in an electrically conductive liquid.

15. The method according to any one of claims 13 to 14, **characterized in that** an electric field is applied by said unit (2) for generating electric pulses with such electric pulses that a voltage increase takes place between the two electrodes (2a, 2b) of 10% to 90% of a target voltage of the electric pulses within a period of 0.1 ns to 1000 ns, the electric pulses have a pulse duration of 5 ns to 50000 ns, and the electric pulses, upon reaching the target voltage, have an electric field strength of 0.5 kV/cm to 100 kV/cm.
